# EUROPEAN PATENT SPECIFICATION

(11) **EP 3 200 768 B1**
(45) Date of publication and mention of the grant of the patent: **02.12.2020**
(21) Application number: 15781987.1
(22) Date of filing: 28.09.2015
(51) Int. Cl.: A61K 9/10, A61K 9/50, A61K 47/14, A61K 31/4439, A61P 1/04, A61K 31/4184

(54) **A SUSPENSION**
SUSPENSION
SUSPENSION

(30) Priority: 29.09.2014 GB 201417169
(43) Date of publication of application: 09.08.2017
(73) Proprietor: Veriton Pharma Limited, Surrey KT13 0YF (GB)
(72) Inventor: MARCH, Graham, Weybridge Surrey KT13 0YF (GB); TITTERSHILL, Andrew, Weybridge Surrey KT13 0YF (GB); SOCORRO, Antonio, Weybridge Surrey KT13 0YF (GB)
(74) Representative: Hutter, Anton
(86) International application number: PCT/GB2015/052803
(87) International publication number: WO 2016/051145

(56) References cited:
- WO-A1-2004/004690
- WO-A1-2005/004921
- WO-A1-2014/167342
- WO-A2-2004/004682

## Description

The present invention relates to suspensions, and more particularly, to stable suspensions of a benzimidazole or a derivative thereof, which is suitable for oral administration. Most particularly, the invention relates to a formulation comprising Omeprazole.

Omeprazole, Pantoprazole, Lansoprazole and other derivatives of benzimidazole are active proton pump inhibitors which decrease gastric secretion and as such are used in the treatment of, for example, dyspepsia, peptic ulcer disease, gastritus, gastroesophageal reflux disease, laryngopharyngeal reflux, gastric and duodenum ulceration and Zollinger-Ellison syndrome. These benzimidazoles are proton pump inhibitors that suppress gastric acid secretion by specific inhibition of the H+/K+-ATPase in the gastric parietal cell. Although the present invention is applicable to the derivatives of benzimidazole selected from Omeprazole, Pantoprazole, Rabeprazole or Lansoprazole, for ease of reference much of the discussion will be given with reference to Omeprazole. However, it should be construed as also being a reference to the alternative derivatives.

Omeprazole, otherwise known as 5-methoxy-2((4-methoxy-3,5-dimethyl-2-pyridinyl)methylsulfinyl)-1H-benzimidazole, is one of the most widely prescribed drugs. In some countries, it is also available "over the counter".

While Omeprazole is usually taken orally, in some countries it may also be provided in injectable form in a combination pack comprising a vial of powder and a separate ampoule of a reconstituting solution.

It is much more common to provide Omeprazole as tablets or capsules. Whilst, a formulation which can be administered orally may offer various advantages, finding a liquid formulation of omeprazole for oral administration has proven problematic as, on contact with acid, degradation occurs. Since the stomach is a highly acidic environment, if the Omeprazole comes into contact with the stomach contents it degrades. Omeprazole degrades with a half-life of less than 10 minutes in an environment with pH values below 4.0. At pH 6.5, the half life of omeprazole is 18 hours and at pH 11 about 300 days.

One solution used is to provide the Omeprazole in a core together with alkaline constituents within an enteric coating to protect it from acid. Accordingly, Omeprazole is most commonly provided as enteric-coated granules located within capsules or tablets. An enteric coating is a polymer coating barrier which is applied to the granules to protect the Omeprazole from the acid in the stomach. Patients who struggle with swallowing tablets are advised to mix the granules from a capsule in a spoonful of suitable carrier, such as apple sauce. The resulting mixture can then be swallowed. However, it has to be taken immediately. Alternatively or additionally, these patients may be provided with a powder for oral suspension which has to be mixed with water. However, the amount of water has to be carefully controlled and the mixture has to be allowed to thicken. The resultant thick suspension has to be taken within about 30 minutes and any excess cannot be stored for future use. Unfortunately, some people find this thick suspension unpleaseant to take. In addition, the requirements for preparing the suspension can be difficult for the patient particularly if they are away from home. These difficulties may result in the patient not following the regimen.

A further problem with asking the patient to mix the granules with apple sauce or the like is that the granules can become damaged if the patient is over vigorous in the mixing. Whilst providing Omeprazole as a suspension is desirable, and some suspensions are made to order in pharmacies or specialist manufacturing facilities, these have a very short shelf-life. It is therefore not currently possible to batch manufacture a suspension with an acceptable shelf-life.

Additionally, since Omeprazole is provided in the form of microgranules these will often sink to the bottom of any suspension, resulting in inconsistency when measuring a dose of the suspension. It would be possible to prevent this by increasing the viscosity of the solvent the microgranules were suspended in. However, if the viscosity is too high then it will be very difficult to pour the suspension from its container and measure the correct dose.

WO 2004/004682 discloses a suspension comprising microgranules of benzimidazole derivatives in a liquid vehicle which comprises medium chain glycerides. The microparticles have a particle size that allow them to remain in suspension.

It is therefore desirable to provide a liquid suspension of benzimidazole or a derivative thereof (e.g. Omeprazole), which is stable such that the patient can readily take the required dosage as a liquid without having to mix powders and the like.

In accordance with a first aspect of the invention, there is provided a suspension comprising microgranules of a benzimidazole derivative selected from Omeprazole, Pantoprazole, Rabeprazole or Lansoprazole, or a mixture thereof suspended in an organic solvent, wherein the average diameter of the microgranules is 100 to 900 µm and wherein at least 40 % of the microgranules have a diameter in the range of 500 to700 µm and less than 10 % of the microgranules in the suspension have a diameter of more than 900 µm, as measured by a Malvern Mastersizer, and wherein the suspension has a standing viscosity of 4000 to 7000 mPa·s (centipoise) at 15 to 25 °C, wherein the standing viscosity is measured after the suspension has been left to stand for 5 minutes or more, and the viscosity of the suspension is 3000 to 4000 mPa·s (centipoise) at 15 to 25 °C when shaken, wherein the shaken viscosity is measured after the suspension has been shaken vigorously to cause turbulent flow of the suspension within a container.

Advantageously, the high standing viscosity of the suspension results in the microgranules remaining fully suspended in solution, thereby ensuring homogeneity for the measuring of a dose when it is administered to a subject. However, the reduction in viscosity when the suspension is shaken allows a portion of the suspension to be poured from its container and measured when a dose is administered. Thus, the suspension of the invention provides an elegant solution to the problem of ensuring that the viscosity is sufficiently high to retain accuracy of dosages and yet sufficiently low such that the suspension can be poured and thereby administered to a subject.

The suspension comprises a benzimidazole derivative selected from Omeprazole, Pantoprazole, Rabeprazole and/or Lansoprazole. The suspension may comprise a mixture of suitable benzimidazole derivatives. Where the derivative has optically active forms, it may be provided as the racemate or as one of the resolved isomers. Preferably, however, the benzimidazole derivative is Omeprazole.

The benzimidazole derivative may be in the form of enteric coated beads. In a preferred embodiment, however, the microgranules comprise cores with the benzimidazole derivative disposed on the surface thereof. Any suitable biocompatible core may be used as a substrate for the benzimidazole derivative. In a preferred embodiment, the core comprises sugar.

The microgranules may comprise an enteric coating, which may comprise a single coating layer. However, preferably the enteric coating comprises a plurality of coating layers. The enteric coating may comprise a layer comprising a disintegrant, a sealing layer and/or a control release layer. The order in which the layers are applied is considered to be important to the performance of the enteric coated granule.

Disintegrants are agents which promote the breakup of a pellet or microgranule into smaller fragments. The layer comprising the disintergrant may comprise one or more disintergrants selected from a list consisting of: pre-gellatinized starch, microcrystalline cellulose, sodium bicarbonate, alginic acid, an ion exchange resin, modified starches, such as sodium carboxymethyl starch, sodium starch glycolate or modified cellulose, such as sodium carboxymethyl cellulose. Preferably, the layer comprising the disintergrant comprises sodium starch glycolate.

The sealing layer may comprise cellulose, such as hydroxypropyl methylcellulose, microcrystalline cellulose, polyvinylpyrrolidone, hydroxymethylcellulose, hydroxyethylcellulose or dextran. Preferably, the sealing layer comprises hydroxypropyl methylcellulose.

The control release layer may be pH and/or time-dependent. The layer may be formed from methacrylic acid and/or ethyl acrylate or copolymers thereof. Preferably, the control release later comprises methacrylic acid.

The enteric coating may comprise an emulsifier, detackifier, surfactant, flow aid, flavourant and/or colourant.

In one embodiment, the microgranules in the suspension have an average diameter of 100 to 900 µm. Preferably, the microgranules in the suspension have an average diameter of 200 to 900 µm, more preferably 300 to 900 µm, even more preferably 400 to 800 µm, and still more preferably 500 to 700 µm. Most preferably, the microgranules in the suspension have an average diameter of 600 to 700 µm. Even more preferably, the average diameter of the microgranules is between about 620 and 660 µm.

In a preferred embodiment, the average diameter of the microgranules in the suspension is normally distributed. At least 40% of the microgranules in the suspension have a diameter in the range of 500 to 700 µm. Preferably, at least 50%, 60% or 70% of the microgranules in the suspension have a diameter in the range of 500 to 700 µm, especially 600 to 700 µm.

Preferably, less that 30% of the microgranules in the suspension have a diameter in the range of 300 to 500 µm. More preferably, less than 25%, 20% or 15% of the microgranules in the suspension have a diameter in the range of 300 to 500 µm.

Preferably, less than 10 %, 5% or 1% of the microgranules in the suspension have a diameter of less than 300 µm.

Preferably, less that 30% of the microgranules in the suspension have a diameter in the range of 700 to 900 µm. More preferably, less than 25%, 20% or 15% of the microgranules in the suspension have a diameter in the range of 700 to 900 µm.

Less than 10% of the microgranules in the suspension have a diameter of more than 900 µm. Preferably, less than 5% or 1% of the microgranules in the suspension have a diameter of more than 900 µm.

The microgranules may comprise from about 4% to about 15% by weight of the benzimidazole derivative with the remainder being made of the core and/or coating. In one arrangement, the microgranules may comprise from about 6% to about 12% by weight, preferably about 7% to about 10% by weight of the benzimidazole derivative.

The organic solvent may comprise an ester of an acid or an ester of a fatty acid. The ester may comprise a triglyceride ester or a propylene glycol ester. Alternatively, or additionally, the ester may be formed with a fatty acid or mixture of acids. The fatty acid may be derived from coconut or palm kernel oil. In one embodiment, a mixture of triglyceride esters of fatty acids is used. The chain length of the fatty acid may be selected to provide the desired viscosity of the finished suspension. In one arrangement, the triglyceride ester component may be a mixture of triglyceride esters of C₈ to C₁₀ fatty acids. One example of a suitable triglyceride ester is that which is sold under the trade name Mygliol BP/USP. Mygliol 812 N may also be used.

The term "standing viscosity" is the viscosity of the suspension as measured after the suspension has been left to stand for 5 minutes or more.

The term "viscosity when shaken" is the viscosity of the suspension as measured immediately after a container containing the suspension has been shaken vigorously by a user. The skilled person will appreciate that vigorous shaking is carried out for a sufficient length of time (e.g. 3 seconds to 20 seconds or more) in order to cause turbulent flow of the suspension within its container.

As mentioned above, the viscosity of the suspension will change (i.e. decrease) when it is shaken. The suspension is therefore thixotropic. During manufacture it will be necessary to mix the suspension. Accordingly, it will be understood that the viscosity of the suspension immediately after mixing will differ from the standing viscosity of the suspension and/or the viscosity of the suspension when it is shaken. Additionally, during manufacture, the solution is mixed prior to the addition of the microgranules.

Accordingly, the solution prior to the addition of the microgranules will also have a different viscosity to the solution once the granules have been added.

In a preferred embodiment, the solution in the absence of microgranules has a viscosity of less than 2000 mPa·s (cp) at 15 to 25°C immediately after being mixed in the manufacturing process. Preferably, the solution in the absence of microgranules has a viscosity of between 1000 to 2000 mPa·s (cp), and more preferably about 1500 mPa·s (cp) at 15 to 25°C immediately after being mixed in the manufacturing process.

In a preferred embodiment, the suspension has a viscosity of between 1500 and 3500 mPa·s (cp) at 15 to 25°C immediately after the solution has been mixed in the manufacturing process and the microgranules have been added thereto. Preferably, the suspension has a viscosity of 2000 to 3000 mPa·s (cp), and more preferably between 2000 and 2600 mPa·s (cp) at 15 to 25°C immediately after the solution has been mixed in the manufacturing process and the microgranules have been added thereto.

In a preferred embodiment, the viscosity of the suspension increases on standing for a number of weeks and stabilizes between 4000 and 7000 mPa·s (cp) at 15 to 25°C. Preferably, the viscosity of the suspension stabilizes between 4500 and 6000 mPa·s (cp) at 15 to 25°C.

The viscosity of the suspension is between 3000 and 4000 mPa·s (cp) at 15 to 25°C immediately after the suspension has been shaken by a user. Shaking may be quantified by vigorous movement of the container back and forth.

Preferably, the suspension comprises a stabiliser. The stabiliser may be selected from a list consisting of: benzoic acid, calcium propoanate, potassium hydrogen sulphite, sodium nitrite, and silicon dioxide. Preferably, the stabiliser is silicon dioxide.

Additionally, or alternatively, a combination of two or more stabilisers may be used. The amount of stabiliser present in the suspension may be from about 1% (w/v) to about 5% (w/v), preferably about 2% (w/v) to about 3% (w/v).

Preferably, the suspension comprises a preservative. The preservative may have antimicrobial properties. The preservative may be selected from a list consisting of: potassium sorbate, propylene glycol, disodium, edentate, butylparaben, sodium benzoate, and methyl paraben. Preferably, the preservative is potassium sorbate. The amount of preservative present in the suspension may be from about 0.1% (w/v) to about 0.5% (w/v), preferably about 0.2% (w/v) to about 0.3% (w/v).

Preferably, the suspension comprises a sweetener. The sweetener may be selected from a list consisting of: zylose, ribose, glucose, mannose, fructose, dextrose, sucrose, maltose, sorbitol, mannitol, glycerin, corn syrup, sodium glucamate, sodium saccharin, aspartame, and mixtures thereof. Preferably, the sweetener is saccharin. The amount of sweetener may be selected to give the desired level of sweetness. For example, the amount of sweetener present in the solution may be about 0.05% (w/v) to about 0.2% (w/v).

Preferably, the solution comprises a flavouring. Preferably, the flavouring is peppermint oil. Alternatively, a fruit flavouring may be used. The amount of flavouring present in the suspension may be selected to achieve an acceptable flavouring. The amount of flavouring present in the solution may be about 0.05% (w/v) to about 0.2% (w/v).

The suspension may also include a rafting agent, which offers additional benefits in the treatment of the gastric acid problems. One suitable rafting agent is alginic acid. The rafting agent may be present in any suitable amount but may be present in from about 5% (w/v) to about 15% (w/v) and may be present in about 10% (w/v).

The components of the present invention will generally be of sufficiently high purity and sufficiently low toxicity to render them suitable for administration to the patient.

Preferably, the suspension is stable for at least 1 month, more preferably at least 2 months, more preferably at least 3 months, more preferably at least 4 months, more preferably at least 5 months, still more preferably at least 6 months. A product will generally be regarded as being no longer stable when an assay of the formulation shows that content of the active pharmaceutical ingredient has fallen below 90% of the stated label claim, i.e. if the formulation is 20mg of active in 5ml the assay shows that the active content has fallen below 18mg in 5ml. Stability can also be defined in terms of impurities and related substances. In the case of Omeprazole it is 0.5% of the stated amount of the active.

The amount of the benzimidazole derivative present in the suspension will depend on the strength of final suspension required. Examples of suitable amounts will be 5 mg in 5 ml, 10 mg in 5ml, 20 mg in 5 ml, 30 mg in 5 ml, 40 mg in 5 ml, 50 mg in 5 ml, 60 mg in 5 ml, 70 mg in 5 ml and 80 mg in 5 ml.

In accordance with a second aspect, there is provided a suspension in accordance with the first aspect, for use as a medicament or in therapy.

It will be understood that the suspension of the first aspect is particularly suitable as an active proton pump inhibitor which decreases gastric secretion and as such may be used in the treatment of, for example, dyspepsia, peptic ulcer disease, gastritis, gastroesophageal reflux disease, laryngopharyngeal reflux, gastric and duodenum ulceration and Zollinger-Ellison syndrome.

Accordingly, in accordance with a third aspect there is provided a suspension in accordance with the first aspect, for use as an active proton pump inhibitor which decreases gastric secretion.

Preferably, the suspension is for use in the treatment, prevention or amelioration of dyspepsia, peptic ulcer disease, gastritis, gastroesophageal reflux disease, laryngopharyngeal reflux, gastric and duodenum ulceration and/or Zollinger-Ellison syndrome.

In accordance with a fourth aspect there is provided a method of treating, preventing or ameliorating dyspepsia, peptic ulcer disease, gastritis, gastroesophageal reflux disease, laryngopharyngeal reflux, gastric and duodenum ulceration or Zollinger-Ellison syndrome, the method comprising administering a suspension in accordance with the first aspect to a subject in need thereof.

The suspension is preferably administered to a patient orally.

It will be appreciated that the amount of the suspension that is required is determined by its biological activity and bioavailability, which in turn depends on the mode of administration, the physiochemical properties of the benzimidazole derivative, and whether it is being used as a monotherapy, or in a combined therapy. The frequency of administration will also be influenced by the half-life of the compound within the patient being treated. Optimal dosages to be administered may be determined by those skilled in the art, and will vary with the particular compound in use, the strength of the pharmaceutical composition, the mode of administration, and the advancement of the dyspepsia, peptic ulcer disease, gastritis, gastroesophageal reflux disease, laryngopharyngeal reflux, gastric and duodenum ulceration or Zollinger-Ellison syndrome. Additional factors depending on the particular subject being treated will result in a need to adjust dosages, including subject age, weight, gender, diet, and time of administration.

Generally, a daily dose of the suspension comprising between 0.01µg/kg of body weight and 500mg/kg of body weight of the benzimidazole derivative may be used for treating, ameliorating, or preventing dyspepsia, peptic ulcer disease, gastritis, gastroesophageal reflux disease, laryngopharyngeal reflux, gastric and duodenum ulceration or Zollinger-Ellison syndrome depending upon which compound is used. More preferably, the daily dose comprises between 0.01mg/kg of body weight and 400mg/kg of body weight, more preferably between 0.1mg/kg and 200mg/kg body weight, and most preferably between approximately 1mg/kg and 100mg/kg body weight of the benzimidazole derivative.

The suspension may be administered before, during or after onset of the dyspepsia, peptic ulcer disease, gastritis, gastroesophageal reflux disease, laryngopharyngeal reflux, gastric and duodenum ulceration or Zollinger-Ellison syndrome to be treated. Daily doses may be given as a single administration. Alternatively, the dyspepsia, peptic ulcer disease, gastritis, gastroesophageal reflux disease, laryngopharyngeal reflux, gastric and duodenum ulceration or Zollinger-Ellison syndrome may require administration twice or more times during a day. As an example, a suspension according to the first aspect may be administered as two (or more depending upon the severity of the dyspepsia, peptic ulcer disease, gastritis, gastroesophageal reflux disease, laryngopharyngeal reflux, gastric and duodenum ulceration or Zollinger-Ellison syndrome being treated) daily doses comprising between 5mg and 7000 mg (i.e. assuming a body weight of 70 kg) of the benzimidazole derivative. A patient receiving treatment may take a first dose upon waking and then a second dose in the evening (if on a two dose regime) or at 3- or 4-hourly intervals thereafter.

A "subject" maybe a vertebrate, mammal, or domestic animal. Hence, suspensions may be used to treat any mammal, for example livestock (e.g. a horse), pets, or may be used in other veterinary applications. Most preferably, however, the patient is a human being.

A "therapeutically effective amount" of suspension is any amount which, when administered to a subject, is the amount of drug that is needed to treat the target disease, or produce the desired effect, i.e. decreases gastric secretion.

For example, the therapeutically effective amount of compound used may be from about 0.01 mg to about 800 mg, and preferably from about 0.01 mg to about 500 mg. It is preferred that the amount of compound is an amount from about 0.1 mg to about 250 mg, and most preferably from about 0.1 mg to about 20 mg.

All features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying Figures, in which:-
**Figure 1** is a table showing the stability of Omeprazole 10mg/5ml at 25°C with a relative humidity of 60%;
**Figure 2** is a table showing the stability of Omeprazole 10mg/5ml at 40°C with a relative humidity of 75%;
**Figure 3** is a table showing the stability of Omeprazole 20mg/5ml at 25°C with a relative humidity of 60%;
**Figure 4** is a table showing the stability of Omeprazole 20mg/5ml at 40°C with a relative humidity of 75%; and
**Figure 5** shows a cross-section of an embodiment of a microgranule of a benzimidazole derivative suspended in an organic solvent.

### Example 1 - Microgranules

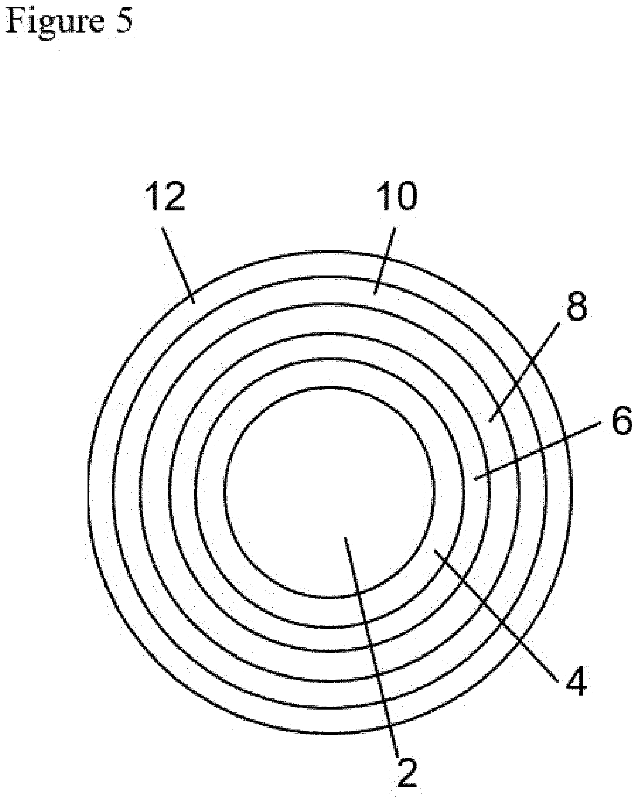

With reference to Figure 5, enteric coated microgranules 2 comprising 8.5% by weight omeprazole were formed by coating a sugar core 4 with Omeprazole 6, and then providing enteric layers comprising sodium starch glycolate 8, hydroxypropyl methylcellulose 10 and methacrylic acid 12 thereon. The sugar core 4 provides a substrate on which to build the microgranule 2 from. The omeprazole 6 is the active agent in the microgranule 2, and the sodium starch glycolate 8 is provided to act as a disintegrant. The hydroxypropyl methylcellulose 10 is provided to comprise a sealing layer between the Omeprazole 6 and the solution. The methacrylic acid 12 is provided to comprise a control release layer. The outer layer 12 is pH dependent.

The omeprazole microgranules 2 were measured on a Malvern Mastersizer, and the average diameter of the granules was approximately 640 µm.

The microgranule sizes exhibited a normal distribution bell curve with approximately 69% of the granules having a diameter between 500 and 700 µm, approximately 15.5% of the granules having a diameter between 700 and 900 µm and approximately 15.5% of the granules having a diameter between 300 and 500 µm.

A solution was formed by:-
1. The saccharin is mixed into a proportion of the (heated)Myglyol. The mixture is then allowed to cool.
2. The potassium sorbate, titanium dioxide, and mint are then added and mixed.
3. The silicon dioxide is then added and mixed.
4. Finally, the Omeprazole beads are added and mixed.

Potassium sorbate acts as a preservative, saccarin acts as a sweetener, silicon dioxide acts as a stabilizer and enhances the viscosity, peppermint oil is added for flavouring, titanium dioxide is added to give the suspension a white colour and the fractionated coconut oil is the organic solvent.

A first suspension comprised 10mg of omeprazole microgranules 2 for every 5ml of the suspension, and a second suspension comprised 20mg of omeprazole microgranules 2 for every 5ml of the suspension. The quantities of the constituents present in each suspension are given below.

A first sample of each suspension was maintained at 25°C with a relative humidity of 60% and a second sample of each suspension was maintained at 40°C with a relative humidity of 75%. Characteristics of the samples were checked after the suspensions had been manufactured two weeks after manufacture, as well as at various time points thereafter.

Due to the presence of the titanium dioxide all of the samples initially appeared as a white suspension. The appearance was checked visually at each time point. Due to the presence of the peppermint oil, all of the samples initially smelled of mint. The odour of the samples was checked manually at each time point.

The viscosity of each sample was measured by rheometer.

Once the viscosity of a sample had been checked, 5 samples of 5ml were taken from the initial sample. Each 5ml sample was analysed to record the quantity of omeprazole present in the sample using a HPLC based analytical method. The mean of the five samples was then calculated. The first suspension was deemed to comply if the recorded mean was between 9.00 to 11.00 mg of omeprazole in 5ml of the sample and the second suspension was deemed to comply if the recorded mean was between 18.00 to 22.00 mg of omeprazole in 5ml of the sample.

The samples were checked for related but known substances using a HPLC analytical method. The samples were deemed to comply where there was no more than (NMT) 0.2% by area of unknown substances and no more than 0.5% by area of known substances. "RRT" refers to Relative Retention Time (of the peak(s)).

While not tested at each time point, the microbial purity of the samples was also checked. A sample was placed onto growing medium on plates and then incubated. The sample was deemed to comply if:- (i) the total viable count (TVC) of colony forming units (cfu) was no more than 10,000 cfu/g, (ii) the total fungal counts (TFC) is no more than 1,000 cfu/g, and (iii) if *E. coli* was absent in 1 gram of the sample.

### Example 2 - Omeprazole (10mg/5ml)

The first suspension comprised the following quantities of the various constituents.

**Omeprazole microganules (as 3.53 grams per Example 1)**

| | |
|---|---|
| Potassium sorbate | 360 milligrams |
| Saccarin | 180 milligrams |
| Silicon dioxide | 5.67 grams |
| Peppermint oil | 180 milligrams |
| Titanium dioxide | 900 milligrams |
| Fractionated coconut oil | To make up to 200ml |
| (Sasol® Miglyol 812N) | (approximately 146 grams) |

The mixture was filled up to 150mL.

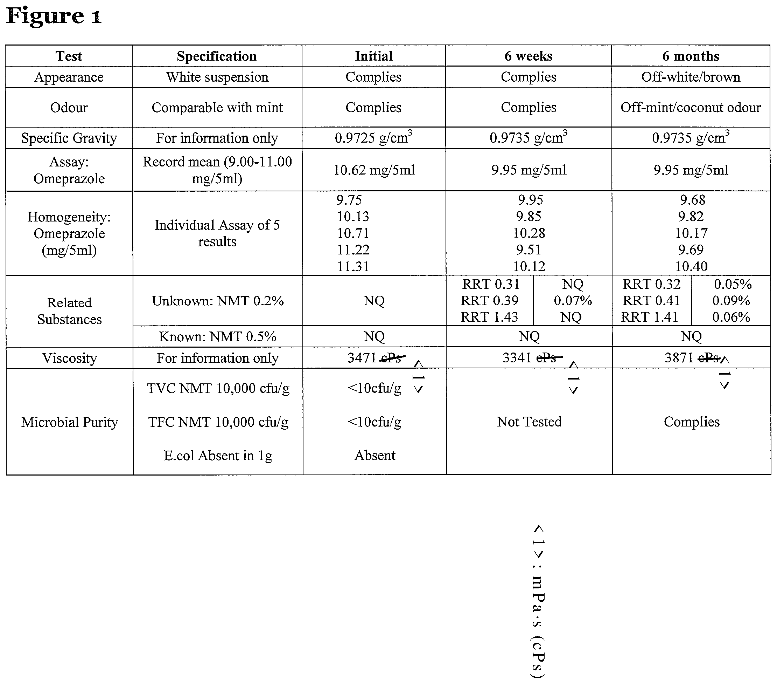

The characteristics of the first sample of the first suspension were maintained at 25°C with a relative humidity of 60%, and were checked initially, and then at 6 weeks and 6 months after the initial check, and the results are shown in Figure 1.

It will be noted that the suspension complied with all the checks and was therefore stable after being maintained for 6 months under the above conditions. Additionally, while the appearance and odour of the suspension had deteriorated at the 6 month test, the suspension was still microbially pure, the related substances in the solution did not exceed the predetermined maximum and the microgranules 2 were still dispersed throughout the suspension such that each dose was homogeneous. Accordingly, the suspension would still be a safe and effective treatment if used at this point.

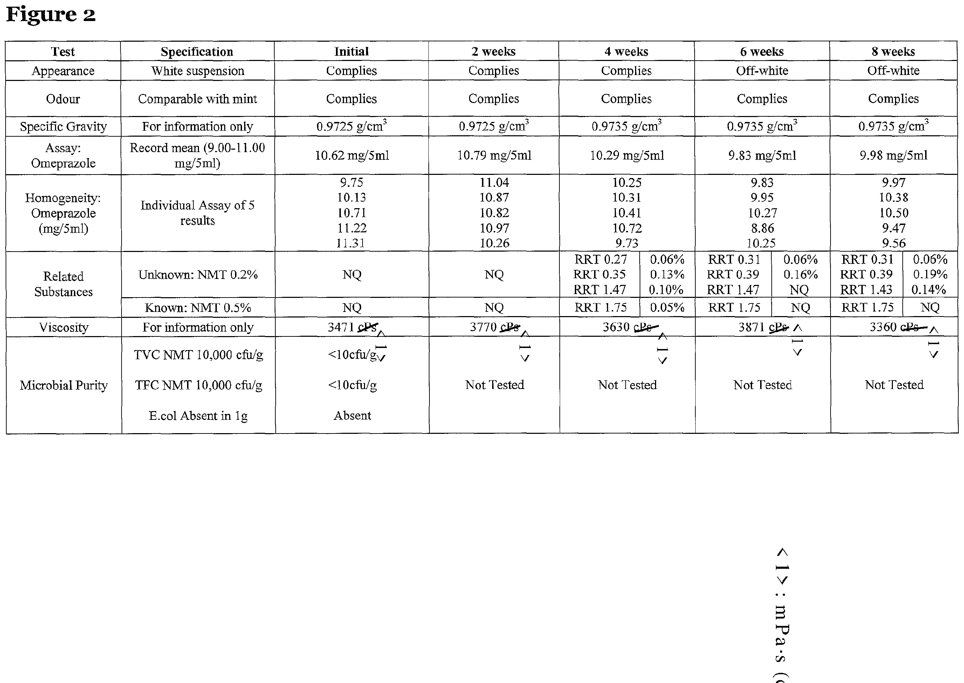

The same characteristics of the second sample maintained at 40°C with a relative humidity of 75% were checked initially, as well as at 2 weeks, 4 weeks, 6 weeks and 8 weeks after the initial check, and the results are shown in Figure 2.

It will be noted that the suspension complied with all the checks and was therefore stable after being maintained for 4 weeks under the above conditions. Additionally, while the appearance of the suspension had deteriorated at the 6 week test, at both the 6 and 8 week tests the suspension was still microbially pure, the related substances in the suspension did not exceed the predetermined maximum and the microgranules 2 were still dispersed throughout the suspension such that each dose was homogeneous. Accordingly, the suspension would still be a safe and effective treatment if used at this point.

### Example 3 - Omeprazole (20mg/5ml)

The second suspension comprised the following quantities of the various constituents.

**Omeprazole microganules (as 7.06 grams per Example 1)**

| | |
|---|---|
| Potassium sorbate | 360 milligrams |
| Saccarin | 180 milligrams |
| Silicon dioxide | 5.67 grams |
| Peppermint oil | 180 milligrams |
| Titanium dioxide | 900 milligrams |
| Fractionated coconut oil | To make up to 200ml |
| (Sasol® Miglyol 812N) | (approximately 143 grams) |

The mixture was filled up to 150mL.

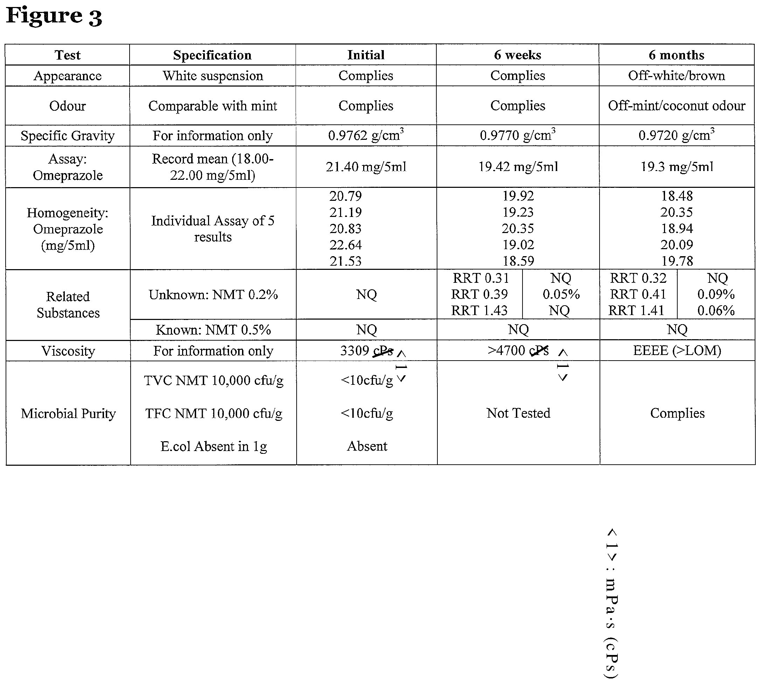

The characteristics of the first sample of the second suspension maintained at 25°C with a relative humidity of 60% were checked initially, as well as at 6 weeks and 6 months after the initial check, and the results are shown in Figure 3.

It will be noted that the suspension complied with all the checks and was therefore stable after being maintained for 6 weeks under the above conditions. As with the first sample of the first suspension, while the appearance and odour of the suspension had deteriorated at the 6 month test the suspension was still microbially pure, the related substances in the solution did not exceed the predetermined maximum and the microgranules 2 were still dispersed throughout the suspension such that each dose was homogeneous. Accordingly, the suspension would still be a safe and effective treatment if used at this point. "EEEE (>LOM)" refers to Error, greater than limit of measurement'.

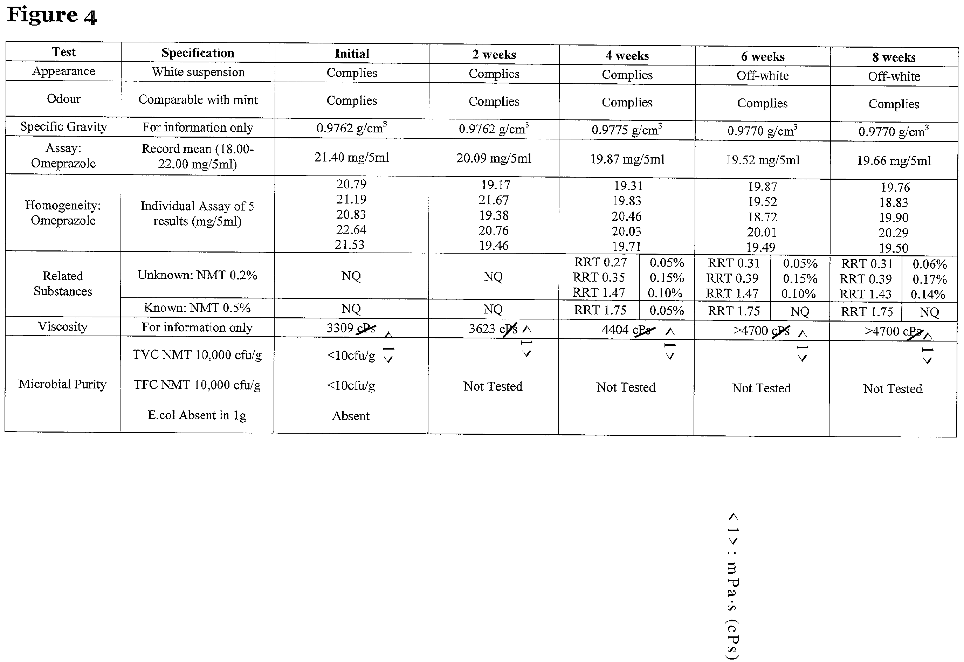

The same characteristics of the second sample maintained at 40°C with a relative humidity of 75% were checked initially, as well as at 2 weeks, 4 weeks, 6 weeks and 8 weeks after the initial check, and the results are shown in Figure 4.

It will be noted that the suspension complied with all the checks and was therefore stable after being maintained for 4 weeks under the above conditions. Additionally, while the appearance of the suspension had deteriorated by the 6 week test, at both the 6 and 8 week tests the related substances in the suspension did not exceed the predetermined maximum and the microgranules 2 were still dispersed throughout the suspension such that each dose was homogeneous. Accordingly, the suspension would still be a safe and effective treatment if used at this point.

### Example 4 - Viscosity

| **Sample** | **Initial (25 July 2014)** | **08-Aug-14** |
|---|---|---|
| Post Manufacture | 1136.94 | 3458.63 |
| 15 Min Gate | 1420.01 | 3588.45 |
| 30 Min Gate | 1592.94 | 4031.32 |
| 45 Min Gate | 1566.69 | 4129.27 |
| 60 Min Gate | 1666.05 | 3834.96 |
| 75 Min Gate | 1717.13 | 4183.04 |
| 90 Min Gate | 1998.32 | 4319.39 |
| 105 Min Gate | 1910.21 | 4353.76 |

The readings for the 25 July 2014 are on samples measured on the day of manufacture. The readings taken on 08 August 2014 are the samples tested two weeks later. The '15 min gate', '30 min gate', '45 min gate' are mixing times of the excipients but without Omeprazole beads being added. This table shows two things: (1) that the longer the mixing time on the day of manufacture, the greater the viscosity, and (2) that at all mixing times the viscosity increases after manufacture over the subsequent two weeks. This study helped determine that a mixing time of circa 15 minutes was the optimum mixing time.

### Summary

Advantages of the suspension of the invention reside in the ability to provide a stable ready made suspension which can be stored and administered as necessary. Additionally, the viscosity characteristics of the suspension allow a person to pour and measure a desired volume of suspension from a container in confidence that the desired volume will contain a consistent dose.

## Claims

1. A suspension comprising microgranules of a benzimidazole derivative selected from Omeprazole, Pantoprazole, Rabeprazole or Lansoprazole, or a mixture thereof suspended in an organic solvent, wherein the average diameter of the microgranules is 100 to 900 µm and wherein at least 40% of the microgranules have a diameter in the range of 500 to 700 µm and less than 10 % of the microgranules in the suspension have a diameter of more than 900 µm, and wherein the suspension has a standing viscosity of 4000 to 7000 mPa·s (centipoise) at 15 to 25°C, wherein the standing viscosity is measured after the suspension has been left to stand for 5 minutes or more, and the viscosity of the suspension is 3000 to 4000 mPa·s at 15 to 25°C when shaken, wherein the shaken viscosity is measured after the suspension has been shaken vigorously to cause turbulent flow of the suspension within a container.

2. A suspension according to claim 1, wherein the benzimidazole derivative is Omeprazole and/or is in the form of enteric coated beads.

3. A suspension according to any preceding claim, wherein the microgranules comprise cores with the benzimidazole derivative disposed on the surface thereof, preferably wherein the core comprises sugar.

4. A suspension according to any preceding claim, wherein the microgranules comprise an enteric coating, which preferably comprises:
- a layer comprising a disintegrant, which is preferably selected from a list consisting of: pre-gellatinized starch, microcrystalline cellulose, sodium bicarbonate, alginic acid, an ion exchange resin, modified starches, such as sodium carboxymethyl starch, sodium starch glycolate or modified cellulose, such as sodium carboxymethyl cellulose;
- a sealing layer, preferably wherein the sealing layer comprises cellulose, polyvinylpyrrolidone, hydroxymethylcellulose, hydroxyethylcellulose or dextran, and most preferably wherein the sealing layer comprises hydroxypropyl methylcellulose; and/or
- a control release layer, preferably wherein the control release layer is pH and/or time-dependent, more preferably wherein the control release layer is formed from methacrylic acid and/or ethyl acrylate or copolymers thereof.

5. A suspension according to claim 4, wherein the enteric coating comprises an emulsifier, detackifier, surfactant, flow aid, flavourant and/or colourant.

6. A suspension according to any preceding claim, wherein:
- the microgranules in the suspension have an average diameter of 200 to 900 µm, or 300 to 900 µm, or 400 to 800 µm, or 500 to 700 µm, or 600 to 700 µm, or 620 and 660 µm, as measured by a Malvern Mastersizer;
- at least 50%, 60% or 70% of the microgranules in the suspension have a diameter in the range of 500 to 700 µm, especially 600 to 700 µm, as measured by a Malvern Mastersizer;
- less than 30%, 25%, 20% or 15% of the microgranules in the suspension have a diameter in the range of 300 to 500 µm, as measured by a Malvern Mastersizer;
- less than 10 %, 5% or 1% of the microgranules in the suspension have a diameter of less than 300 µm, as measured by a Malvern Mastersizer;
- less that 30%, 25%, 20% or 15% of the microgranules in the suspension have a diameter in the range of 700 to 900 µm, as measured by a Malvern Mastersizer; and/or
- less than 5% or 1% of the microgranules in the suspension have a diameter of more than 900 µm, as measured by a Malvern Mastersizer.

7. A suspension according to any preceding claim, wherein the microgranules comprise from 4% to 15% by weight of the benzimidazole derivative with the remainder being made of the core and/or coating, preferably wherein the microgranules comprise from 6% to 12% by weight, or 7% to 10% by weight of the benzimidazole derivative.

8. A suspension according to any preceding claim, wherein the organic solvent comprises an ester of an acid or an ester of a fatty acid, wherein:
- the ester comprises a triglyceride ester or a propylene glycol ester; or
- the ester is formed with a fatty acid or mixture of acids, preferably wherein the fatty acid is derived from coconut or palm kernel oil; and/or
- a mixture of triglyceride esters of fatty acids is used, and/or wherein the triglyceride ester component is a mixture of triglyceride esters of C₈ to C₁₀ fatty acids.

9. A suspension according to any preceding claim, wherein the solution in the absence of microgranules has a viscosity of less than 2000 mPa·s at 15 to 25°C, or between 1000 and 2000 mPa·s, or about 1500 mPa·s at 15 to 25°C immediately after being mixed in the manufacturing process, and/or wherein the suspension has a viscosity of between 1500 and 3500 mPa·s at 15 to 25°C, or 2000 to 3000 mPa·s at 15 to 25°C, or between 2000 and 2600 mPa·s at 15 to 25°C immediately after the solution has been mixed in the manufacturing process and the microgranules have been added thereto.

10. A suspension according to any preceding claim, wherein the viscosity of the suspension increases on standing for a number of weeks and stabilizes between 4000 and 7000 mPa·s (cp) at 15 to 25°C, or between 4500 and 6000 mPa·s (cp) at 15 to 25°C, and/or wherein the viscosity of the suspension is between 3000 and 4000 mPa·s (cp) at 15 to 25°C immediately after the suspension has been shaken by a user.

11. A suspension according to any preceding claim, wherein the suspension comprises:
- a stabiliser, which is selected from a list consisting of: benzoic acid, calcium propoanate, potassium hydrogen sulphite, sodium nitrite, and silicon dioxide, preferably wherein the amount of stabiliser present in the suspension is from about 1% (w/v) to about 5% (w/v), or about 2% (w/v) to about 3% (w/v);
- a preservative, which is selected from a list consisting of: potassium sorbate, propylene glycol, disodium, edentate, butylparaben, sodium benzoate, and methyl paraben, preferably wherein the amount of preservative present in the suspension is from about 0.1% (w/v) to about 0.5% (w/v), or about 0.2% (w/v) to about 0.3% (w/v);
- a sweetener, which is selected from a list consisting of: zylose, ribose, glucose, mannose, fructose, dextrose, sucrose, maltose, sorbitol, mannitol, glycerin, corn syrup, sodium glucamate, sodium saccharin, aspartame, and mixtures thereof, preferably wherein the amount of sweetener present in the solution is about 0.05% (w/v) to about 0.2% (w/v); and/or
- a flavouring, which is peppermint oil or a fruit flavouring, preferably wherein the amount of flavouring present in the solution is about 0.05% (w/v) to about 0.2% (w/v).

12. A suspension according to any one of claims 1-11, for use as a medicament or in therapy.

13. A suspension according to any one of claims 1-11, for use as an active proton pump inhibitor which decreases gastric secretion.

14. A suspension for use according to claim 13, for use in the treatment, prevention or amelioration of dyspepsia, peptic ulcer disease, gastritis, gastroesophageal reflux disease, laryngopharyngeal reflux, gastric and duodenum ulceration and/or Zollinger-Ellison syndrome.

## Patentansprüche

1. Suspension, umfassend Mikrokörnchen eines Benzimidazol-Derivats, ausgewählt aus Omeprazol, Pantoprazol, Rabeprazol oder Lansoprazol oder einem Gemisch davon, suspendiert in einem organischen Lösungsmittel, wobei der durchschnittliche Durchmesser der Mikrokörnchen 100 bis 900 µm ist und wobei mindestens 40 % der Mikrokörnchen einen Durchmesser in dem Bereich von 500 bis 700 µm aufweisen und weniger als 10 % der Mikrogranulate in der Suspension einen Durchmesser von mehr als 900 µm aufweisen, und wobei die Suspension eine Stehviskosität von 4000 bis 7000 mPa·s (Centipoise) bei 15 bis 25 °C aufweist, wobei die Stehviskosität gemessen wird, nachdem die Suspension 5 Minuten oder länger stehen gelassen wurde, und die Viskosität der Suspension 3000 bis 4000 mPa·s bei 15 bis 25 °C ist, wenn sie geschüttelt wird, wobei die Schüttelviskosität gemessen wird, nachdem die Suspension kräftig geschüttelt wurde, um eine turbulente Strömung der Suspension in einem Behälter zu bewirken.

2. Suspension nach Anspruch 1, wobei das Benzimidazol-Derivat Omeprazol ist und/oder in Form von magensaftresistent beschichteten Kügelchen ist.

3. Suspension nach einem der vorherigen Ansprüche, wobei die Mikrokörnchen Kerne umfassen, auf deren Oberfläche das Benzimidazol-Derivat aufgebracht ist, vorzugsweise wobei der Kern Zucker umfasst.

4. Suspension nach einem der vorherigen Ansprüche, wobei die Mikrokörnchen eine magensaftresistente Beschichtung aufweisen, die vorzugsweise Folgendes umfasst:
- eine Schicht, umfassend ein Sprengmittel, das vorzugsweise ausgewählt ist aus einer Liste, bestehend aus: vor-gelatinierter Stärke, mikrokristalliner Cellulose, Natriumbicarbonat, Alginsäure, einem Ionenaustauscherharz, modifizierten Stärken, wie z. B. Natriumcarboxymethylstärke, Natriumstärkeglycolat oder modifizierter Cellulose, wie z. B. Natriumcarboxymethylcellulose;
- einer Abdichtungsschicht, vorzugsweise wobei die Abdichtungsschicht Cellulose, Polyvinylpyrrolidon, Hydroxymethylcellulose, Hydroxyethylcellulose oder Dextran umfasst und am meisten bevorzugt wobei die Abdichtungsschicht Hydroxypropylmethylcellulose umfasst; und/oder
- eine Schicht für kontrollierte Freisetzung, vorzugsweise wobei die Schicht für kontrollierte Freisetzung pH- und/oder zeitabhängig ist, bevorzugter wobei die Schicht für kontrollierte Freisetzung aus Methacrylsäure und/oder Ethylacrylat oder Copolymeren davon gebildet ist.

5. Suspension nach Anspruch 4, wobei die magensaftresistente Beschichtung einen Emulgator, Klebrigmacher, ein Tensid, Fließhilfsmittel, einen Aromastoff und/oder Farbstoff umfasst.

6. Suspension nach einem vorherigen Anspruch, wobei:
die Mikrokörner in der Suspension einen durchschnittlichen Durchmesser von 200 bis 900 µm, oder 300 bis 900 µm, oder 400 bis 800 µm, oder 500 bis 700 µm, oder 600 bis 700 µm, oder 620 und 660 µm, gemessen mit einem Malvern Mastersizer, aufweisen;
mindestens 50 %, 60 % oder 70 % der Mikrokörnchen in der Suspension einen Durchmesser in dem Bereich von 500 bis 700 µm, insbesondere 600 bis 700 µm, gemessen mit einem Malvern Mastersizer, aufweisen;
weniger als 30 %, 25 %, 20 % oder 15 % der Mikrokörnchen in der Suspension einen Durchmesser in dem Bereich von 300 bis 500 µm, gemessen mit einem Malvern Mastersizer, aufweisen;
weniger als 10 %, 5 % oder 1 % der Mikrokörnchen in der Suspension einen Durchmesser von weniger als 300 µm, gemessen mit einem Malvern Mastersizer, aufweisen;
weniger der 30 %, 25 %, 20 % oder 15 % der Mikrokörnchen in der Suspension einen Durchmesser in dem Bereich von 700 bis 900 µm, gemessen mit einem Malvern Mastersizer, aufweisen; und/oder
weniger als 5 % oder 1 % der Mikrokörnchen in der Suspension einen Durchmesser von mehr als 900 µm, gemessen mit einem Malvern Mastersizer, aufweisen.

7. Suspension nach einem der vorherigen Ansprüche, wobei die Mikrokörnchen 4 bis 15 Gewichtsprozent des Benzimidazol-Derivats umfassen und der Rest aus dem Kern und/oder der Beschichtung besteht, wobei die Mikrokörnchen vorzugsweise 6 bis 12 Gewichtsprozent oder 7 bis 10 Gewichtsprozent des Benzimidazol-Derivats umfassen.

8. Suspension nach einem der vorherigen Ansprüche, wobei das organische Lösungsmittel einen Ester einer Säure oder einen Ester einer Fettsäure umfasst, wobei:
- der Ester einen Triglyceridester oder einen Propylenglykolester umfasst; oder
- der Ester mit einer Fettsäure oder einem Gemisch von Säuren gebildet wird, vorzugsweise wobei die Fettsäure von Kokos- oder Palmkernöl abgeleitet ist; und/oder
- ein Gemisch von Triglyceridestern von Fettsäuren verwendet wird, und/oder wobei die Triglyceridester-Komponente ein Gemisch von Triglyceridestern von C₈ bis C₁₀-Fettsäuren ist.

9. Suspension nach einem der vorherigen Ansprüche, wobei die Lösung in Abwesenheit von Mikrokörnchen eine Viskosität von weniger als 2000 mPa·s bei 15 bis 25 °C oder zwischen 1000 und 2000 mPa·s oder etwa 1500 mPa·s bei 15 bis 25 °C unmittelbar nach dem Mischen in dem Herstellungsverfahren aufweist, und/oder wobei die Suspension eine Viskosität zwischen 1500 und 3500 mPa·s bei 15 bis 25 °C oder 2000 bis 3000 mPa·s bei 15 bis 25 °C oder zwischen 2000 und 2600 mPa·s bei 15 bis 25 °C unmittelbar nach dem Mischen der Lösung in dem Herstellungsverfahren und dem Hinzufügen der Mikrogranulate dazu aufweist.

10. Suspension nach einem der vorherigen Ansprüche, wobei die Viskosität der Suspension bei Stehen über eine Anzahl von Wochen ansteigt und sich zwischen 4000 und 7000 mPa·s (cp) bei 15 bis 25 °C oder zwischen 4500 und 6000 mPa·s (cp) bei 15 bis 25 °C stabilisiert, und/oder wobei die Viskosität der Suspension zwischen 3000 und 4000 mPa·s (cp) bei 15 bis 25 °C unmittelbar nachdem die Suspension von einem Anwender geschüttelt wurde, ist.

11. Suspension nach einem vorherigen Anspruch, wobei die Suspension Folgendes umfasst:
- einen Stabilisator, der ausgewählt ist aus einer Liste, bestehend aus: Benzoesäure, Calciumpropoanat, Kaliumhydrogensulfit, Natriumnitrit und Siliciumdioxid, vorzugsweise wobei die Menge des in der Suspension vorhandenen Stabilisators von etwa 1 % (Gewicht/Volumen) bis etwa 5 % (Gewicht/Volumen) oder etwa 2 % (Gewicht/Volumen) bis etwa 3 % (Gewicht/Volumen) ist;
- ein Konservierungsmittel, das ausgewählt ist aus einer Liste, bestehend aus: Kaliumsorbat, Propylenglykol, Dinatrium, Edentat, Butylparaben, Natriumbenzoat und Methylparaben, vorzugsweise wobei die Menge des in der Suspension vorhandenen Konservierungsmittels von etwa 0,1 % (Gewicht/Volumen) bis etwa 0,5 % (Gewicht/Volumen) oder etwa 0,2 % (Gewicht/Volumen) bis etwa 0,3 % (Gewicht/Volumen) ist;
- einen Süßstoff, der ausgewählt ist aus einer Liste, bestehend aus: Zylose, Ribose, Glucose, Mannose, Fructose, Dextrose, Saccharose, Maltose, Sorbit, Mannit, Glycerin, Maissirup, Natriumglucamat, Natriumsaccharin, Aspartam und Gemischen davon, vorzugsweise wobei die Menge des in der Lösung vorhandenen Süßstoffs etwa 0,05 % (Gewicht/Volumen) bis etwa 0,2 % (Gewicht/Volumen) ist; und/oder
- ein Aroma, das Pfefferminzöl oder ein Fruchtaroma ist, vorzugsweise wobei die Menge des in der Lösung vorhandenen Aromas etwa 0,05 % (Gewicht/Volumen) bis etwa 0,2 % (Gewicht/Volumen) ist.

12. Suspension nach einem der Ansprüche 1-11, zur Verwendung als Medikament oder in Therapie.

13. Suspension nach einem der Ansprüche 1-11 zur Verwendung als aktiver Protonenpumpenhemmer, der Magensekretion verringert.

14. Suspension nach Anspruch 13 zur Verwendung bei der Behandlung, Prävention oder Linderung von Dyspepsie, Magengeschwüren, Gastritis, gastroösophagealer Refluxkrankheit, laryngopharyngealem Reflux, Magen- und Zwölffingerdarmgeschwüren und/oder Zollinger-Lison-Syndrom.

## Revendications

1. Suspension comprenant des microgranules d'un dérivé de benzimidazole choisi parmi l'oméprazole, le pantoprazole, le rabéprazole ou le lansoprazole, ou un mélange de ceux-ci mis en suspension dans un solvant organique, le diamètre moyen des microgranules étant 100 à 900 µm et au moins 40 % des microgranules présentant un diamètre dans la plage de 500 à 700 I1m et moins de 10 % des microgranules dans la suspension présentant un diamètre supérieur à 900 µm, et ladite suspension présentant une viscosité au repos de 4000 à 7000 mPa·s (centipoise) à 15 à 25°C, ladite viscosité au repos étant mesurée après avoir laissé au repos la suspension pendant 5 minutes ou plus, et ladite viscosité de la suspension étant 3000 à 4000 mPa·s à 15 à 25°C lorsqu'elle est agitée, la viscosité après agitation étant mesurée après agitation vigoureuse de la suspension pour provoquer un écoulement turbulent de la suspension dans un récipient.

2. Suspension selon la revendication 1, ledit dérivé de benzimidazole étant l'oméprazole et/ou étant sous la forme de billes à enrobage entérique.

3. Suspension selon l'une quelconque des revendications précédentes, lesdits microgranules comprenant des noyaux, le dérivé de benzimidazole étant disposé à la surface de ceux-ci, de préférence ledit noyau comprenant du sucre.

4. Suspension selon l'une quelconque des revendications précédentes, lesdits microgranules comprenant un enrobage entérique, qui de préférence comprend :
- une couche comprenant un délitant, qui est de préférence choisi dans une liste constituée par : l'amidon prégélatinisé, la cellulose microcristalline, le bicarbonate de sodium, l'acide alginique, une résine échangeuse d'ions, les amidons modifiés tels que le carboxyméthylamidon de sodium, le glycolate d'amidon sodique ou une cellulose modifiée telle que la carboxyméthylcellulose de sodium ;
- une couche de scellement, de préférence ladite couche de scellement comprenant de la cellulose, de la polyvinylpyrrolidone, de l'hydroxyméthylcellulose, de l'hydroxyéthylcellulose ou du dextrane, et idéalement ladite couche de scellement comprenant de l'hydroxypropylméthylcellulose ; et/ou
- une couche de libération contrôlée, de préférence ladite couche de libération contrôlée étant fonction du pH et/ou du temps, plus préférablement ladite couche de libération contrôlée étant formée d'acide méthacrylique et/ou d'acrylate d'éthyle ou de copolymères de ceux-ci.

5. Suspension selon la revendication 4, ledit revêtement entérique comprenant un émulsifiant, un anti-adhésif, un tensioactif, un fluidifiant, un arôme et/ou un colorant.

6. Suspension selon l'une quelconque des revendications précédentes :
- lesdits microgranules dans la suspension présentant un diamètre moyen de 200 à 900 µm, ou 300 à 900 µm, ou 400 à 800 µm, ou 500 à 700 µm, ou 600 à 700 µm, ou 620 et 660 µm, tel que mesuré par un granulomètre Malvern Mastersizer ;
- au moins 50 %, 60 % ou 70 % des microgranules dans la suspension présentant un diamètre dans la plage de 500 à 700 µm, en particulier 600 à 700 µm, tel que mesuré par un granulomètre Malvern Mastersizer ;
- moins de 30%, 25%, 20% ou 15% des microgranules dans la suspension présentant un diamètre dans la plage de 300 à 500 µm, tel que mesuré par un granulomètre Malvern Mastersizer ;
- moins de 10 %, 5 % ou 1 % des microgranules dans la suspension présentant un diamètre inférieur à 300 µm, tel que mesuré par un granulomètre Malvern Mastersizer ;
- moins de 30%, 25%, 20% ou 15% des microgranules dans la suspension présentant un diamètre dans la plage de 700 à 900 µm, tel que mesuré par un granulomètre Malvern Mastersizer ; et/ou
- moins de 5 % ou 1 % des microgranules dans la suspension présentant un diamètre supérieur à 900 µm, tel que mesuré par un granulomètre Malvern Mastersizer.

7. Suspension selon l'une quelconque des revendications précédentes, lesdits microgranules comprenant de 4 % à 15 % en poids du dérivé de benzimidazole, le reste étant constitué du noyau et/ou de l'enrobage, de préférence lesdits microgranules comprenant de 6 % à 12 % en poids, ou 7 % à 10 % en poids du dérivé de benzimidazole.

8. Suspension selon l'une quelconque des revendications précédentes, ledit solvant organique comprenant un ester d'un acide ou un ester d'un acide gras :
- ledit ester comprenant un ester de triglycéride ou un ester de propylène glycol ; ou
- ledit ester étant formé avec un acide gras ou un mélange d'acides, de préférence ledit acide gras provenant d'huile de palmiste ou de noix de coco ; et/ou
- un mélange d'esters de triglycéride d'acides gras étant utilisé, et/ou ledit composant d'ester de triglycéride étant un mélange d'esters de triglycéride d'acides gras en C₈ à C₁₀.

9. Suspension selon l'une quelconque des revendications précédentes, ladite solution en l'absence de microgranules présentant une viscosité inférieure à 2000 mPa·s à 15 à 25°C, ou comprise entre 1000 et 2000 mPa·s, ou d'environ 1500 mPa·s à 15 à 25°C immédiatement après avoir été mélangée dans le processus de fabrication, et/ou ladite suspension présentant une viscosité comprise entre 1500 et 3500 mPa·s à 15 à 25°C, ou 2000 à 3000 mPa·s à 15 à 25°C, ou comprise entre 2000 et 2600 mPa·s à 15 à 25°C immédiatement après mélange de la solution dans le processus de fabrication et ajout des microgranules à celle-ci.

10. Suspension selon l'une quelconque des revendications précédentes, ladite viscosité de la suspension augmentant au repos pendant un certain nombre de semaine et se stabilisant entre 4000 et 7000 mPa·s (cp) à 15 à 25°C, ou entre 4500 et 6000 mPa·s (cp) à 15 à 25°C, et/ou ladite viscosité de la suspension étant comprise entre 3000 et 4000 mPa·s (cp) à 15 à 25°C immédiatement après agitation de la suspension par un utilisateur.

11. Suspension selon l'une quelconque des revendications précédentes, ladite suspension comprenant :
- un stabilisant qui est choisi dans une liste constituée par : l'acide benzoïque, le propoanate de calcium, l'hydrogénosulfite de potassium, le nitrite de sodium et le dioxyde de silicium, de préférence la quantité de stabilisant présente dans la suspension allant d'environ 1 % (p/v) à environ 5 % (p/v), ou d'environ 2 % (p/v) à environ 3 % (p/v) ;
- un conservateur qui est choisi dans la liste constituée par : le sorbate de potassium, le propylène glycol, le disodium, l'édentate, le butylparabène, le benzoate de sodium et le méthylparabène, de préférence la quantité de conservateur présente dans la suspension allant d'environ 0,1 % (p/v) à environ 0,5 % (p/v), ou d'environ 0,2 % (p/v) à environ 0,3 % (p/v) ;
- un édulcorant qui est choisi dans la liste constituée par : le zylose, le ribose, le glucose, le mannose, le fructose, le dextrose, le saccharose, le maltose, le sorbitol, le mannitol, la glycérine, le sirop de maïs, le glucamate de sodium, la saccharine de sodium, l'aspartame et les mélanges de ceux-ci, de préférence la quantité d'édulcorant présente dans la solution étant d'environ 0,05 % (p/v) à environ 0,2 % (p/v) ; et/ou
- un arôme qui est de l'huile de menthe poivrée ou un arôme de fruit, de préférence la quantité d'arôme présente dans la solution étant d'environ 0,05 % (p/v) à environ 0,2 % (p/v).

12. Suspension selon l'une quelconque des revendications 1 à 11, pour utilisation comme médicament ou en thérapie.

13. Suspension selon l'une quelconque des revendications 1 à 11, pour utilisation comme inhibiteur de pompe à protons actif qui réduit la sécrétion gastrique.

14. Suspension pour utilisation selon la revendication 13, pour utilisation dans le traitement, la prévention ou l'amélioration de la dyspepsie, d'une pathologie d'ulcère peptique, la gastrite, une pathologie de reflux gastro-œsophagien, le reflux laryngo-pharyngé, un ulcère de l'estomac et du duodénum et/ou le syndrome de Zollinger-Ellison.
